# EUROPEAN PATENT APPLICATION

(11) **EP 3 805 148 A1**
(43) Date of publication of application: **14.04.2021**
(21) Application number: 19810093.5
(22) Date of filing: 14.05.2019
(51) Int. Cl.: C01B 3/26, B01J 8/02, B01J 23/42, C01B 3/22, C07C 5/367, C07C 15/06, C07C 15/073, C07C 15/24, C07C 45/29, C07C 47/055, C07C 47/06, C07C 49/08, C07D 213/133, C07D 217/02, C07B 61/00

(54) **DEVICE AND CATALYST FOR USE WITH SAME**

(30) Priority: 29.05.2018 JP 2018102465
(71) Applicant: SAIDA FDS INC., Yaizu-shi Shizuoka 4250054 (JP); N.E. Chemcat Corporation, Tokyo 1056124 (JP)
(72) Inventor: YOSHIMURA, Takeo, Yaizu-shi Shizuoka 4250054 (JP); OHNEDA, Noriyuki, Yaizu-shi Shizuoka 4250054 (JP); SAJIKI, Hironao, Gifu-shi, Gifu 5011113 (JP); MONGUCHI, Yasunari, Fukuoka-shi, Fukuoka 8150037 (JP); SAWAMA, Yoshinari, Gifu-shi, Gifu 5011113 (JP); YAMADA, Tsuyoshi, Gifu-shi, Gifu 5011113 (JP); ICHIKAWA, Tomohiro, Gifu-shi, Gifu 5011113 (JP); MATSUO, Tomohiro, Gifu-shi, Gifu 5011113 (JP); TACHIKAWA, Takumu, Gifu-shi, Gifu 5011113 (JP); KOMATSU, Akira, Tokyo 1056124 (JP)
(74) Representative: Blodig, Wolfgang
(86) International application number: PCT/JP2019/019098
(87) International publication number: WO 2019/230368

(57) **Abstract**

Provided is a device including: a storage section in which a solution containing an organic compound can be stored; a catalyst holding section in which a solid catalyst is held; and a microwave irradiation means configured to irradiate the solution passing through the catalyst holding section with a microwave, the device being characterized in that the solid catalyst is a molded catalyst containing a noble metal supported on a carrier that has an average particle diameter larger than 100 µm. Also provided is a hydrogen production method characterized by including irradiating a solution containing an organic compound, the solution passing through a catalyst holding section holding a solid catalyst, with a microwave, the solid catalyst being a molded catalyst containing a noble metal supported on a carrier that has an average particle diameter larger than 100 µm. The device and the method, which do not require a high-temperature heat source or the like, enable easy collection, replacement, or the like of the catalyst, and can be used for production of hydrogen and the like.

## Description

### Technical Field

The present invention relates to a device that can be used for producing hydrogen from a solution containing an organic compound and a catalyst and the like for use in the device.

### Background Art

In techniques known in the related art, hydrogen is produced from a liquid source, such as methanol, by a steam reforming method, or from an aqueous methanol solution by using a dehydrogenation reaction.

However, the production of hydrogen by a steam reforming method requires a high-temperature reaction condition at, typically, 200°C or higher. Thus, a high-temperature heat source or the like is required, leading to problems mainly in safety and efficiency. In the production of hydrogen by using a dehydrogenation reaction in the related art, a homogeneous catalyst is mainly used. Accordingly, it is difficult to collect or replace the catalyst, leading to a problem mainly in continuity (PTL 1, NPL 1).

In another known example, a fuel and water are subjected to a steam reforming reaction on a heterogeneous reforming catalyst, but a high temperature condition is required. (PTLs 2 and 3)

In another known method, hydrogen is produced from an organic hydride by using a heterogeneous catalyst and a microwave (PTL 4). However, there are problems of poor efficiency (high power output) and generation of a hot spot (NPL 2).

### Citation List

### Patent Literature

PTL 1: JP-A-2013-163624
PTL 2: JP-A-2016-130186
PTL 3: WO2013/039091
PTL 4: JP-A-2016-175821

### Non-patent Literature

NPL 1: Chem. Commun., 2015, 51, 6714-6725
NPL 2: Kuriin Enerugii (clean energy), 2017, 7, 15-24

### Summary of Invention

### Technical Problem

Thus, an object of the present invention is to provide a device and a hydrogen generation method that do not require a high-temperature heat source or the like, enable easy collection, replacement, or the like of a catalyst, and can be used for production of hydrogen and the like.

### Solution to Problem

As a result of intensive studies for solving the above problem, the present inventors have found that the above problem can be solved by using a device including a storage section in which a solution containing an organic compound can be stored, a catalyst holding section in which a solid catalyst is held, and a microwave irradiation means configured to irradiate the solution passing through the catalyst holding section with a microwave, the solid catalyst having a specific shape, completing the present invention.

The present inventors also have found that hydrogen produced by the device can be used in hydrogenation or the like, completing the present invention.

Specifically, the present invention relates to a device including
a storage section in which a solution containing an organic compound can be stored,
a catalyst holding section in which a solid catalyst is held, and
a microwave irradiation means configured to irradiate the solution passing through the catalyst holding section with a microwave,
characterized in that the solid catalyst is a molded catalyst containing a noble metal supported on a carrier that has an average particle diameter larger than 100 µm.

The present invention also relates to a catalyst for microwave irradiation, characterized in that the catalyst is a molded catalyst containing a noble metal supported on a carrier that has an average particle diameter larger than 100 µm.

The present invention further relates to a method for producing hydrogen, characterized by including irradiating a solution containing an organic compound, the solution passing through a catalyst holding section that holds a solid catalyst, with a microwave, the solid catalyst being a molded catalyst containing a noble metal supported on a carrier that has an average particle diameter larger than 100 µm.

The present invention further relates to an aromatization method characterized by including irradiating a solution containing an organic compound, the solution passing through a catalyst holding section that holds a solid catalyst, with a microwave, the solid catalyst being a molded catalyst containing a noble metal supported on a carrier that has an average particle diameter larger than 100 µm.

The present invention further relates to a hydrogenation method characterized by including irradiating a solution containing an organic compound, the solution passing through a catalyst holding section that holds a solid catalyst, with a microwave, the solid catalyst being a molded catalyst containing a noble metal supported on a carrier that has an average particle diameter larger than 100 µm.

### Advantageous Effects of Invention

The device of the present invention is configured to irradiate a solution containing an organic compound, the solution passing through a catalyst holding section that holds a solid catalyst, with a microwave, the solid catalyst being a molded catalyst containing a noble metal supported on a carrier that has an average particle diameter larger than 100 µm. Accordingly, the device can quickly increase a temperature to a temperature suitable for producing hydrogen from the solution to thus perform hydrogen production with high safety, high efficiency, and high continuity.

On the other hand, while a powder carrier generally used in such a reaction has a large geometric surface area per weight of a catalyst and thus is expected to have a high activity, the fact that such a high active reaction can be achieved even when using a catalyst having a small geometric surface area per weight of the catalyst, such as the molded carrier of the present invention, is one of notable characteristics of the present invention.

In addition, when a reaction vessel is filled with a molded carrier, voids are formed and a large amount of a reactant can flow therethrough. The fact that a reaction can be promoted with high efficiency by using a catalyst containing a carrier having the above action is very advantageous in the industrial field. In the present invention, a reaction, such as mainly a dehydrogenation reaction, can be promoted at a lower temperature than ever. Such a state of low temperature can be confirmed by observing a catalyst holding vessel in reaction using a thermographic measurement device. Here, a high temperature may occur in some cases in a local part of the catalyst held in the vessel in reaction, but the temperature of the catalyst as a whole in reaction is a lower temperature than ever, and the ability to promote a highly efficient reaction at such a low temperature is very advantageous in the industrial field in terms not only of energy but also of safety.

In addition, the catalyst for microwave irradiation of the present invention, which is a heterogeneous catalyst, can be easily collected and replaced.

Furthermore, since all the hydrogen production method, aromatization method, and hydrogenation method of the present invention use the solid catalyst as described above, the methods can be achieved with a low temperature in the catalyst holding section with high safety, high efficiency, and high continuity.

### Brief Description of Drawings

Fig. 1 shows formulae illustrating examples of hydrogen production reactions in a hydrogen production device and a hydrogen production method according to a first embodiment of the present invention.
Fig. 2 is a schematic diagram illustrating a configuration of the hydrogen production device according to the first embodiment.
Fig. 3 is a diagram of an entire configuration of a microwave device which is a component of the hydrogen production device according to the first embodiment.
Fig. 4 shows schematic diagrams illustrating a configuration of a cavity resonator of the microwave device according to the first embodiment. (A) is a front elevation viewed from the same direction as in Fig. 3, (B) is a left elevation, and (C) is a plan view.
Fig. 5 is a cross section illustrating an example of an installation state of a distribution tube in (an irradiation chamber of) the cavity resonator in the microwave device according to the first embodiment.
Fig. 6 is a schematic diagram illustrating a configuration of a hydrogen production device according to a second embodiment of the present invention.
Fig. 7 is a cross section illustrating an example of an installation state of a distribution tube in (an irradiation chamber of) a cavity resonator in a microwave device according to the second embodiment.
Fig. 8 is a formula illustrating an example of a hydrogen production reaction in the hydrogen production device and a hydrogen production method according to the second embodiment.
Fig. 9 is a table showing experimental results in the second embodiment.
Fig. 10 is a formula illustrating another example of a hydrogen production reaction in the hydrogen production device and the hydrogen production method in the second embodiment.
Fig. 11 is a graph illustrating a relationship between treatment time and hydrogen purity.
Fig. 12 is a schematic diagram illustrating a configuration of a hydrogen production device according to a third embodiment of the present invention.
Fig. 13 is a formula illustrating an example of a hydrogen production reaction in a hydrogen production device and a hydrogen production method according to a fourth embodiment of the present invention.
Fig. 14 is a table showing results of Experiment 12 of the present invention.

### Description of Embodiments

First, an outline of an embodiment of the present invention will be described. This embodiment provides a device and a method which can continuously produce hydrogen from a solution containing an organic compound. The device in this embodiment is a device including a storage section in which a solution containing an organic compound (hereinafter sometimes simply referred to as "solution") can be stored, a catalyst holding section in which a solid catalyst is held, and a microwave irradiation means configured to irradiate the solution passing through the catalyst holding section with a microwave, the solid catalyst being a molded catalyst containing a noble metal supported on a carrier that has an average particle diameter larger than 100 µm. In the device, a solution containing an organic compound, the solution passing through the catalyst holding section that holds a solid catalyst, the solid catalyst being a molded catalyst containing a noble metal supported on a carrier that has an average particle diameter larger than 100 µm, is irradiated with a microwave to cause a hydrogen production reaction to produce hydrogen. The solution may pass through the catalyst holding section only once, but preferably, the solution circulates in the storage section and the catalyst holding section multiple times to repeatedly cause the reaction. Note that the average particle size, as used herein, is based on weight.

The solution containing an organic compound used in the device is a solution containing an organic compound and a solvent. Examples of the solvent here include water, and organic solvents, for example, alcohols, such as methanol, ethanol, and propanol, hexane, cyclohexane, methylcyclohexane, benzene, and toluene. The organic compound may also function as a solvent. In addition, the solution may contain a basic reagent for increasing the opportunity of contact with the solid catalyst. Examples of the basic reagent include sodium hydroxide and potassium hydroxide, and sodium hydroxide is preferred.

The organic compound is not particularly limited as long as hydrogen is generated through the above reaction, and examples thereof include an alcohol and an organic hydride. Main specific examples of the alcohol include linear or branched lower alkyl alcohols. Among such alcohols, methanol, ethanol, and isopropanol are preferred, and methanol is particularly preferred due to the high hydrogen content. Specific examples of the organic hydride include: saturated hydrocarbons, such as methylcyclohexane, cyclohexane, ethylcyclohexane, methoxycyclohexane, bicyclohexyl, cyclohexylbenzene, and decalin; and heterocyclic compounds, such as tetrahydrofuran (THF), tetrahydropyran (THP), tetrahydroquinoline, tetrahydroisoquinoline, piperidine, methylpiperidine, piperazine, methylpiperazine, and dimethylpiperazine. Among them, methylcyclohexane, ethylcyclohexane, decalin, tetrahydroisoquinoline, methylpiperidine, bicyclohexyl, and cyclohexylbenzene are preferred.

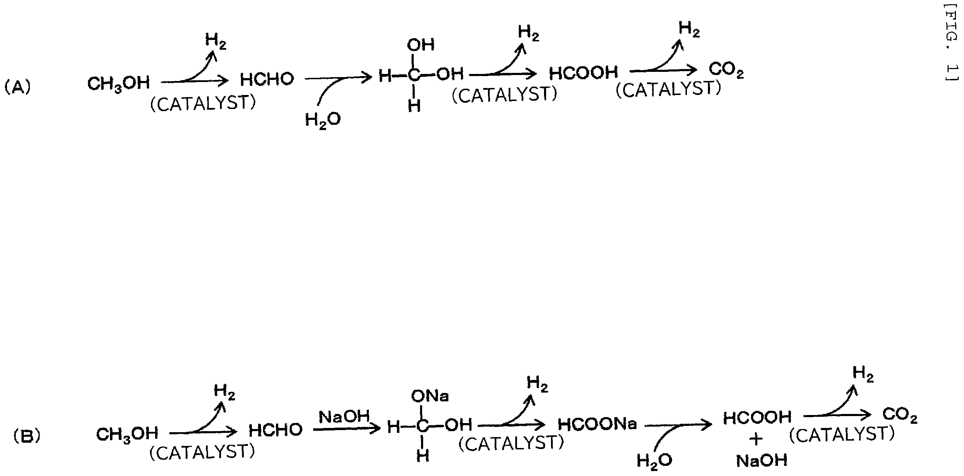

Fig. 1 shows formulae illustrating examples of the hydrogen production reaction in the device and the method according to this embodiment in which the alcohol is methanol and the basic reagent is sodium hydroxide. Fig. 1(A) illustrates a hydrogen production reaction from a mixture solution containing methanol and water (aqueous methanol solution) and Fig. 1(B) illustrates a hydrogen production reaction from a mixture solution containing methanol, water, and sodium hydroxide (aqueous methanol solution + sodium hydroxide).

As illustrated in Fig. 1, when the alcohol is methanol, hydrogen and carbon dioxide can be generated at a ratio of 3:1 from each of a mixture solution containing methanol and water and a mixture solution containing methanol, water, and sodium hydroxide. It is understood that hydrogen (and carbon dioxide) can be continuously produced in each of the hydrogen production reaction shown in Fig. 1(A) and the hydrogen production reaction shown in Fig. 1(B) by appropriately replenishing methanol and water (that is, an aqueous methanol solution) consumed with production of hydrogen (and carbon dioxide) . Of course, sodium hydroxide may be further replenished, as required, in the hydrogen production reaction shown in Fig. 1(B).

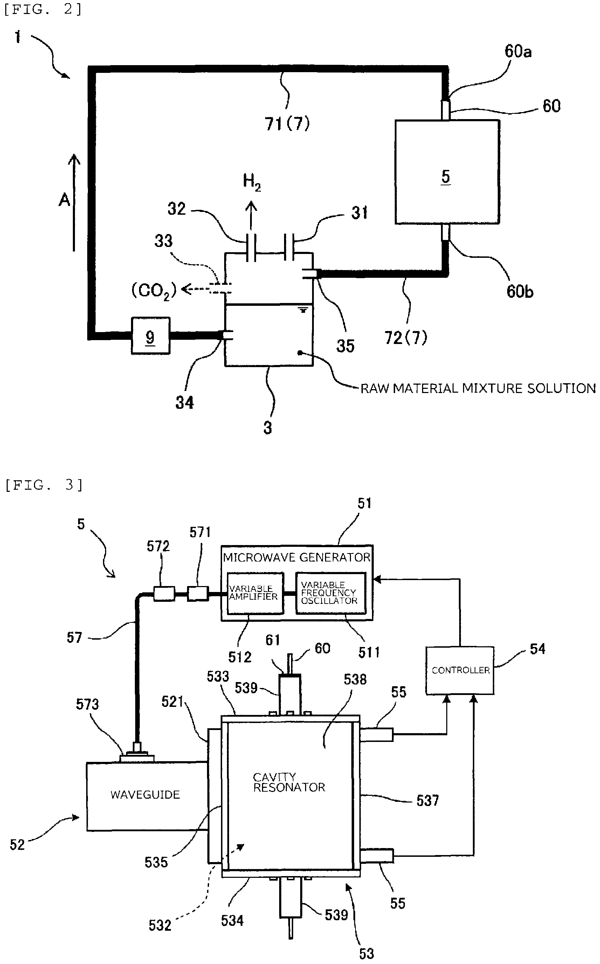

Fig. 2 is a schematic diagram illustrating a configuration of the device according to this embodiment. This device is a hydrogen production device 1 in which hydrogen is produced from a mixture solution containing an alcohol, water, and a basic reagent (hereinafter referred to as "raw material mixture solution"). As illustrated in Fig. 2, the hydrogen production device 1 includes a storage tank 3, a microwave device 5, a fluid passage 7 that couples the storage tank 3 to the microwave device 5, and a liquid feeding pump 9.

The storage tank 3 is capable of storing a prescribed amount of the raw material mixture solution. The storage tank 3 is provided with a supply and replenishment port 31 for supplying and replenishing the raw material mixture solution into the storage tank 3 and a hydrogen takeout port 32 for taking out produced hydrogen. In this embodiment, the supply and replenishment port 31 and the hydrogen takeout port 32 are disposed on the top surface of the storage tank 3. Here, as described above (see Fig. 1), when the alcohol is methanol, carbon dioxide is produced together with hydrogen. Accordingly, when the alcohol is methanol, as illustrated by broken lines in Fig. 2, the storage tank 3 is preferably further provided with a carbon dioxide discharge port 33. In this case, the carbon dioxide discharge port 33 is disposed below the hydrogen takeout port 32 and above the liquid surface of the raw material mixture solution (for example, in an upper portion of a side surface of the storage tank 3).

The microwave device 5 is a resonant cavity-type microwave device. As illustrated in Fig. 3, the microwave device 5 includes a microwave generator 51, a waveguide 52, a cavity resonator 53, and a controller 54. The cavity resonator 53 is provided with an antenna 55 and a distribution tube 60. The microwave device 5 is configured to irradiate a solution to be treated (the raw material mixture solution corresponds to the solution to be treated here) flowing through the distribution tube 60 with a microwave. Components of the microwave device 5 will be described below.

The microwave generator 51 generates a microwave having a prescribed frequency. The microwave generator 51 includes a variable frequency oscillator 511 and a variable amplifier 512. The variable frequency oscillator 511 is capable of outputting a microwave the frequency of which is variable. In this embodiment, in the variable frequency oscillator 511, the frequency of the microwave can be varied in the range of 2.4 GHz to 2.5 GHz which is an ISM frequency band. The variable amplifier 512 amplifies the power of the microwave output from the variable frequency oscillator 511. Note that the operation of the variable frequency oscillator 511 and the variable amplifier 512, that is, the frequency and the power of the microwave output from the microwave generator 51, are controlled by the controller 54.

The waveguide 52 guides a microwave output from the microwave generator 51 toward the cavity resonator 53. Specifically, a microwave output from the microwave generator 51 is sent to a coaxial waveguide converter 573 via an isolator 571, a directional coupler 572, and the like that are coupled via a coaxial cable 57. Then, the microwave passing through the coaxial waveguide converter 573 is guided by the waveguide 52, passes through an iris (binding window, binding slit) 531 to be described later, and is introduced into a cavity (irradiation chamber) 532 formed in the cavity resonator 53.

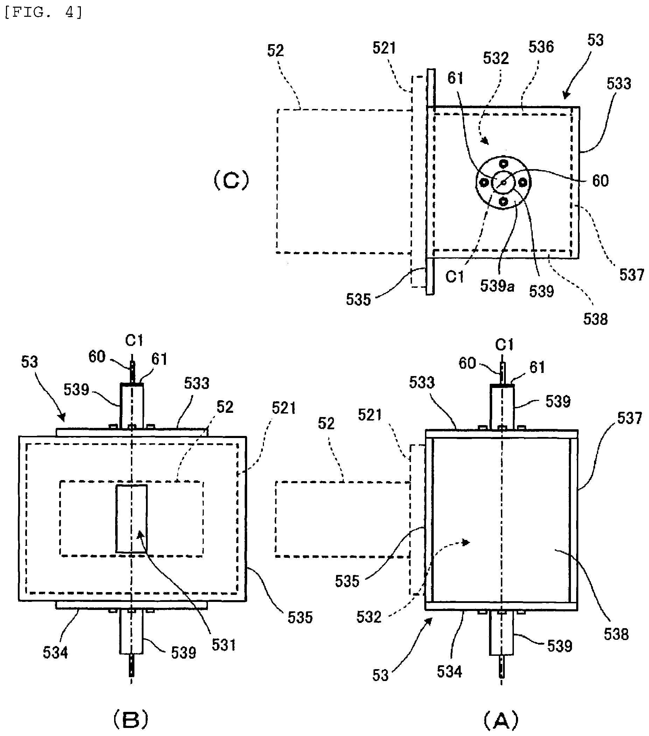

The cavity resonator 53 introduces the microwave into the irradiation chamber 532 to cause resonance in an electromagnetic field. Fig. 4 shows schematic diagrams illustrating a configuration of the cavity resonator 53. Fig. 4(A) is a front elevation of the cavity resonator 53, Fig. 4(B) is a left side elevation of the cavity resonator 53, and Fig. 4(C) is a plan view of the cavity resonator 53.

As shown in Fig. 4, the cavity resonator 53 has a top wall 533 and a bottom wall 534 having a square shape which are opposite to each other and four side walls 535 to 538 having a rectangular shape. The cavity (irradiation chamber) 532 having a square column shape is formed inside the cavity resonator 53. Note that, in this embodiment, the area of the side wall 535 is expanded to correspond to a flange 521 of the waveguide 52 for connecting the waveguide 52.

The iris 531 for introducing the microwave into the irradiation chamber 532 is opened in a central portion of the side wall 535 (Fig. 4 (B)). The iris 531 has a vertically long rectangular shape whose long sides are parallel to a center line C1 of the irradiation chamber 532. The center line C1 of the irradiation chamber 532 is a line joining the center of the top surface of the irradiation chamber 532 and the center of the bottom surface thereof (here, the center of the top wall 533 and the center of the bottom wall 534).

The microwave, which is introduced from the waveguide 52 via the iris 531 into the irradiation chamber 532, generates a single mode electric field along the direction of the center line C1 during resonance. In this embodiment, when there is nothing in the irradiation chamber 532, a TM110 mode electromagnetic field is excited in the irradiation chamber 532. Since the distribution tube 60 and a solution to be treated flowing therethrough exist in the irradiation chamber 532 in practice, the electromagnetic field generated is not a TM110 mode electromagnetic field in a strict sense. However, in a rough sense, an electromagnetic field having a distribution according to a TM110 mode electromagnetic field distribution is generated in the irradiation chamber 532.

The iris 531, which couples the microwave from the waveguide 52 to the cavity resonator 53, involves in making the electromagnetic field exited by the irradiation chamber 532 have only an expected single mode (TM110) . In the iris 531 illustrated in Fig. 4(B), a current caused by the microwave flows in the direction of the center line C1 on the long sides (side edges), and due to the current, a magnetic field surrounding the center line C1 and an electric field parallel to the center line C1 are generated.

When the microwave is introduced into the irradiation chamber 532, the intensity of the electric field or the magnetic field is detected by two antennas 55 (for example, loop antennas) disposed apart from each other in the direction of the center line C1, and the detection results are input into the controller 54. For example, one of the two antenna outputs can be used for observation and the other can be used for control. Note that the two antennas are not necessary and at least an antenna for control is used. In addition, a temperature of the solution to be treated (the raw material mixture solution) detected by a temperature detection unit (not shown) may be input into the controller 54. The controller 54 is configured to control the microwave generator 51 based on the inputs and settings made by an operator.

When an operator performs an operation to start irradiation with a microwave, the controller 54 makes the microwave generator 51 start an output of the microwave to run a frequency control process. This frequency control process is a control to tune the frequency of the microwave output from the microwave generator 51 into the resonance frequency of the irradiation chamber 532 according to a detection result by the antenna 55. The controller 54, when running the frequency control process, determines the tuning frequency based on the detection result by the antenna 55 while sweeping the frequency of the variable frequency oscillator 511.

Following tuning by the frequency control process, the controller 54 runs a power control process to control the power of the microwave. This power control process is a process to control the variable amplifier 512 of the microwave generator 51 according to conditions set by an operator before starting the microwave irradiation, thereby controlling the power of the microwave. In the power control process, the controller 54 adjusts the power of the microwave output from the microwave generator 51 based on a detection result by the antenna 55 and/or a detection result of the temperature of the solution to be treated (the raw material mixture solution).

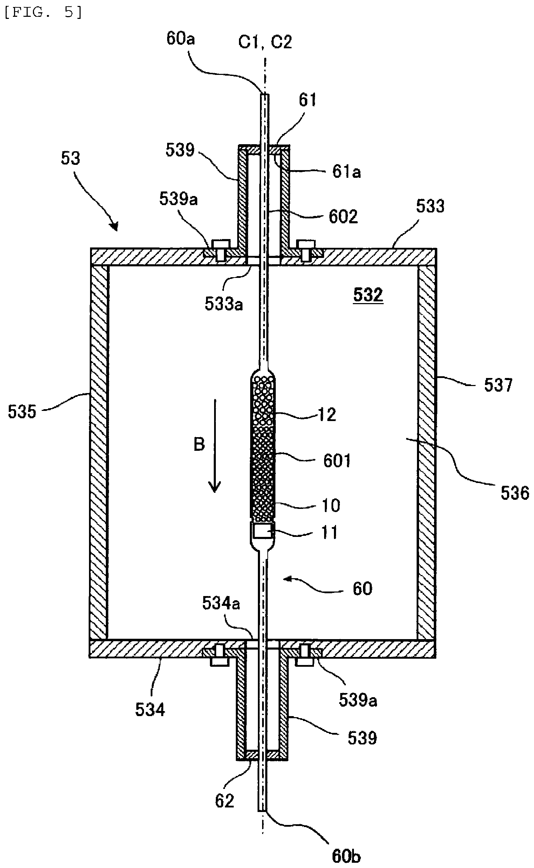

Next, the distribution tube 60 provided in (the irradiation chamber 532 of) the cavity resonator 53 will be described. Fig. 5 is a cross section illustrating an example of an installation state of the distribution tube 60 in the irradiation chamber 532 of the cavity resonator 53. Note that, in this embodiment, the solution to be treated (that is, the raw material mixture solution) flows downwardly from above through the distribution tube 60 (that is, in the irradiation chamber 532) as shown by the arrow B in Fig. 5.

The distribution tube 60 is formed of a material that can transit microwaves and that is resistant to heat generated, and may be, for example, a tube of quartz glass, a ceramic, a resin, or the like formed as a straight tube with an axis (tube axis) C2 of a straight-line shape. The distribution tube 60 has such a length as to pass through the irradiation chamber 532. The distribution tube 60 is disposed so that the axis (tube axis) C2 is almost in line with the center line C1 of the irradiation chamber 532. The center line C1 of the irradiation chamber 532 is in line with the direction of the electric field generated in the irradiation chamber 532, and the electric field is the strongest along the line. Accordingly, by making the axis C2 of the distribution tube 60 almost in line with the center line C1 of the irradiation chamber 532, the solution to be treated (the raw material mixture solution) flowing through the distribution tube 60 can be efficiently irradiated with the microwave. In this embodiment, the distribution tube 60 is detachably attached to the cavity resonator 53 (that is, the irradiation chamber 532).

The installation structure of the distribution tube 60 which is detachably attached to the cavity resonator 53 (the irradiation chamber 532) will be specifically described below.

In this embodiment, in center portions of the top wall 533 and the bottom wall 534 constituting the cavity resonator 53, cylindrical members 539 are each disposed to face the outside. The cylindrical members 539 are configured to support the distribution tube 60 without releasing the microwave introduced in the irradiation chamber 532 to the outside. Specifically, the cylindrical members 539 each have a flange 539a, and the flange 539a is received in a recess formed on the outer surface of each of the top wall 533 and the bottom wall 534 and is fastened thereto with bolts or the like. The center line of the fastened cylindrical member 539 is almost in line with the center line C1 of the irradiation chamber 532, and the interior space of the fastened cylindrical member 539 is in communication with through holes 533a and 534a respectively formed in the centers of the recesses in the top wall 533 and the bottom wall 534.

A lid member 61 is attached to a certain portion on the upper end 60a side of the distribution tube 60. The lid member 61 has a protrusion 61a that is fixed with or threadedly joined to the inner circumferential surface of the cylindrical member 539 which is fastened on the top wall 533.

The distribution tube 60 with the lid member 61 attached to the certain portion of the upper end 60a side thereof is inserted, from the lower end 60b, into the irradiation chamber 532 via (the interior space of) the cylindrical member 539 fastened to the top wall 533 and the through hole 533a formed in the top wall 533. The lower end 60b of the inserted distribution tube 60 passes through the irradiation chamber 532 and extends to the interior space of the cylindrical member 539 fastened to the bottom wall 534. A distribution tube holding member 62 is fixed to the tip end of the cylindrical member 539 fastened to the bottom wall 534. In the distribution tube holding member 62, a through hole having a diameter corresponding to the outer diameter of the distribution tube 60 on the lower end 60b side is formed. Then, with the lower end 60b side of the distribution tube 60 inserted in the through hole of the distribution tube holding member 62, the protrusion 61a of the lid member 61 is fixed with or threadedly joined to the inner circumferential surface of the cylindrical member 539 fastened to the top wall 533. Thus, the distribution tube 60 is installed in (the irradiation chamber 532 of) the cavity resonator 53. Note that the axis C2 of the installed distribution tube 60 is almost in line with the center line C1 of the irradiation chamber 532, and the lower end 60b side of the distribution tube 60 protrudes outside the irradiation chamber 532. The distribution tube 60 can be detached from the cavity resonator 53 by releasing the lid member 61 from the cylindrical member 539 and pulling the distribution tube 60 upward. Accordingly, in this embodiment, replacement or the like of the distribution tube 60 can be achieved easily. Furthermore, the distribution tube 60 may be configured to be held by the lid member 61, and in this case, the distribution tube holding member 62 may be omitted.

The distribution tube 60 will be further described. In this embodiment, the distribution tube 60 includes a catalyst holding section 601 that can hold a solid catalyst 10. In this embodiment, the catalyst holding section 601 has a larger diameter than the other part of the distribution tube 60, and a catalyst holding member 11 is attached to the bottom of the catalyst holding section 601. However, the present invention is not limited to the above configuration, and the catalyst holding section 601 may have the same diameter as the other part of the distribution tube 60. The shape of the catalyst holding section 601 is not particularly limited, and, for example, may be a shape of a straight tube, a straight corrugated tube, or the like. The catalyst holding member 11 allows the solution to be treated (the solution) to pass through therein, while holding the solid catalyst 10 so that the solid catalyst 10 is not dropped. The catalyst holding member 11 is formed of a quartz filter, for example, and is attached to the bottom of the catalyst holding section 601 in production of the distribution tube 60. Alternatively, the catalyst holding member 11 is formed of cotton, glass wool, or the like, and is attached to the bottom of the catalyst holding section 601 before placing the solid catalyst 10 in the catalyst holding section 601. Then, a plurality of (a large number of) solid catalysts 10 are sequentially put from the upper end 60a of the distribution tube 60 and are kept on the catalyst holding member 11 so that the plurality of (the large number of) solid catalysts 10 are held in the catalyst holding section 601.

As described above, the solid catalyst 10 is a molded catalyst containing a noble metal supported on a carrier that has an average particle diameter larger than 100 µm. Examples of the carrier used in the solid catalyst include: inorganic oxides, such as alumina, silica, zeolite, zirconia, titania, and ceria, and composite oxides thereof; carbons, such as activated carbon, carbon nanotube, graphite, and graphene, and carbon compounds, such as silicon carbide and tungsten carbide; and a material containing carbon, such as a material obtained by impregnating the inorganic oxide or the composite oxide with an organic component, such as polyvinyl alcohol or an oil or fat, and steaming the impregnated molded carrier with a carbonization temperature, a calcination time, and a calcination atmosphere adjusted to leave a carbon component. The carriers may be used alone or in combination of two or more thereof. In addition, the carriers can be molded according to an ordinary method into a carrier having an average particle diameter larger than 100 µm. Among the carriers, a carrier containing carbon is preferred, and activated carbon, which has a large specific surface area, which can increase dispersibility of a noble metal to be supported, which is inexpensive, and which has high activity, is more preferred. The source of carbon is not particularly limited, and is preferably, for example, coconut husk fine charcoal or a carbon obtained by petroleum material combustion by an oil furnace method, a Lamp black method, a channel method, a gas furnace method, an acetylene decomposition method, a thermal method, or the like. The carrier can be molded by using a known method, such as a rolling granulation method or extrusion, to produce particles having an average particle size larger than 100 µm. Alternatively, particles having an average particle size larger than 100 µm may be screened after molding. The average particle size of the carrier is larger than 100 µm, preferably 200 µm or larger. The upper limit thereof is not particularly limited, and preferably 10 mm or less, more preferably 1 mm or less, and particularly preferably 500 µm or less. With an average particle size smaller than 100 µm, heat generation due to the microwave absorption is sometimes too large to control the reaction heat or the hot spot. Meanwhile, with an average particle size exceeding 10 mm, the void is too large and the geometric surface area (surface area of sphere) per unit volume of the catalyst is sometimes too small to achieve a sufficient activity when the carrier is used in a catalyst. Note that the particle size of such a carrier may be determined as an average particle size based on weight or as D50 according to a laser diffraction method. The laser diffraction method determines a particle size based on volume, and D50 is a point where the larger side and the smaller side of the particle size distribution are equal in quantity and is also referred to as median size. When the particle size of the carrier used in the present invention is represented by the D50, for the same reason in the average particle size based on weight, D50 measured by a wet method is larger than 100 µm, preferably 200 µm or larger. The upper limit is not particularly limited, and is preferably 2 mm or less, more preferably 1 mm or less, and particularly preferably 500 µm or less. The specific surface area (BET value) of the carrier is not particularly limited, and is, for example, 50 to 2,000 m²/g, preferably 100 to 1,500 m²/g. Note that when the specific surface area is too small, the dispersibility of the noble metal component is reduced to decrease the reactivity in some cases. In addition, although the reason has not been elucidated yet, a too large specific surface area decreases the reactivity in some cases in the method of the present invention. The shape of the carrier is not particularly limited as long as the carrier is molded, and may preferably be a spherical, a columnar, or a pellet shape. The shape is particularly preferably a spherical shape. As such a carrier, besides a carrier produced in the above manner, a commercially available product, such as an activated carbon bead (A-BAC-MP, average particle size: 500 µm, specific surface area: 1200 m²/g, spherical, or A-BAC-SP, average particle size based on weight: 400 µm or less, D50 particle size by a laser diffraction method: 328 µm, specific surface area: 1286 m²/g, spherical) sold by KUREHA CORPORATION, may be used. Note that the average particle size and the specific surface area can be measured according to ordinary methods.

The carrier used in the present invention may be, besides a carrier formed only of the above material, particles that partially contain an inorganic oxide or a composite oxide containing no carbon component which has a significant meaning in the present invention or a resin or glass which does not generate heat by microwaves. The use of such particles can also be said as dilution of the catalyst. When the carrier is diluted with such particles, the size of the particles is not particularly limited and may be larger or smaller than the above carrier. The size can be appropriately selected according to the usage, but preferably is the same size as the above carrier to keep a stable dilution state.

Examples of the noble metal used in the solid catalyst 10 include platinum, palladium, rhodium, ruthenium, and iridium. Among them, platinum or palladium is particularly preferred. The noble metal is preferably contained in an amount of 0.1 to 20% by weight in terms of the metal relative to the carrier, and more preferably contained in an amount of 1 to 10% by weight. Note that, when the amount of the noble metal is too small, the reactivity may decrease, and when the amount is too large, the activity corresponding to the amount may not be obtained. In addition, when the amount of the noble metal is too large, palladium particles on the catalyst may aggregate together, and in this case, the surface area of the entire noble metal particles may decrease to reduce the activity. Besides the noble metal, an additional component may be contained for increasing selectivity to the extent that the reaction proceeds. Examples of the additional component include silver (Ag), nickel (Ni), copper (Cu), cobalt (Co), zinc (Zn), and tin (Sn).

The method for allowing the carrier to support the noble metal is not particularly limited, and examples thereof include an impregnation method, a co-precipitation method, and an adsorption method.

Such a solid catalyst can be easily separated after a reaction and the separated catalyst can be easily reused. In addition, in such a catalyst, since production of a too extreme hot spot to control the reaction by microwave irradiation can be suppressed, the catalyst is considered preferable as a catalyst for microwave irradiation.

Specific examples of the solid catalyst 10 include a "Pt/carbon bead catalyst" containing 0.1 to 15% by weight of platinum supported on a molded spherical activated carbon having an average particle size larger than 100 µm; and a "Pd/carbon bead catalyst" containing 0.1 to 15% by weight of palladium supported on a molded spherical activated carbon having an average particle size larger than 100 µm. The "Pt/carbon bead catalyst" and "Pd/carbon bead catalyst" can be prepared according to a known method or the method described in Examples herein. Another specific example of the solid catalyst 10 is a catalyst containing 0.1 to 15% by weight of platinum or palladium supported on a molded non-spherical activated carbon having an average particle size larger than 100 µm. The catalyst can be prepared according to a known method. Alternatively, a commercially available product, for example, [Pt carbon pellet] (platinum content: 0.5% by weight, columnar, diameter: 2 to 3 mm, length: 3 to 10 mm), [Pd carbon granules (hydrate type)] (palladium content: 0.5% by weight, granular, particle size: 2 to 5 mm), or [Pd carbon granules (W) LA type] (palladium content: 0.5% by weight, granular, particle size: 2 to 5 mm) (all manufactured by N.E. Chemcat Corporation) can be used.

The solid catalyst 10 is present in the catalyst holding section 601 during the reaction, and the amount is, for example, 0.01 to 90 mol%, and preferably 0.1 to 50 mol% relative to the organic compound present in the catalyst holding section 601.

Here, in this embodiment, not only a plurality of the solid catalysts 10 but also a plurality of particulate members 12 are held in the catalyst holding section 601. The plurality of particulate members 12 are disposed above the solid catalysts 10 in the catalyst holding section 601. The carrier as described above or the like may be used as the particulate members 12, or the particulate members 12 may be formed of a material that does not or does almost not absorb microwaves, for example, alumina, fluoride resin, quartz, or borosilicate glass. In this embodiment, the particulate members 12 are larger than the solid catalysts 10. However, the present invention is not limited to the above aspect, and the particulate members 12 may have almost the same size as the solid catalysts 10 or may be smaller than the solid catalysts 10. Then, the plurality of particulate members 12 are disposed above the plurality of solid catalysts 10, for example, by placing the plurality of solid catalysts 10 in the catalyst holding section 601, and then sequentially putting the particulate members 12 from the upper end 60a of the distribution tube 60. The shape of such a catalyst holding section is not particularly limited, and may be a straight tube shape or a spiral shape.

Note that Fig. 5 illustrates an example where the plurality of solid catalysts 10 and the plurality of particulate members 12 each have a spherical shape. However, the present invention is not limited to this aspect, and the solid catalysts 10 and the particulate members 12 may have various shapes, such as a filler shape or a columnar shape.

In addition, a filter material or the like may be disposed between the plurality of solid catalysts 10 and the plurality of particulate members 12. The filter material or the like has flexibility or elasticity, and, for example, the filter material or the like is placed on the plurality of solid catalysts 10 by being inserted from the upper end 60a of the distribution tube 60 after the plurality of solid catalysts 10 are placed in the catalyst holding section 601. Then, after placing the filter material, the plurality of particulate members 12 are placed in the catalyst holding section 601. In such a manner, the plurality of solid catalysts 10 and the plurality of particulate members 12 are securely separated by the filter material or the like, which is convenient particularly when they are collected.

Returning now to Fig. 2, the fluid passage 7 includes a supply passage 71 for guiding the raw material mixture solution in the storage tank 3 to the distribution tube 60 in the microwave device 5 and a return passage 72 for returning the raw material mixture solution flowing out of the distribution tube 60 in the microwave device 5 to the storage tank 3. One end of the supply passage 71 is connected to an outlet 34 for the raw material mixture solution in the storage tank 3, and the other end of the supply passage 71 is connected to the upper end 60a of the distribution tube 60 in the microwave device 5. One end of the return passage 72 is connected to the lower end 60b of the distribution tube 60 in the microwave device 5 and the other end of the return passage 72 is connected to an inlet 35 for the raw material mixture solution in the storage tank 3.

The liquid feeding pump 9 is provided in the supply passage 71. The liquid feeding pump 9 circulates the raw material mixture solution in the order of the storage tank 3 → the supply passage 71 → the distribution tube 60 (the catalyst holding section 601) in the microwave device 5 → the return passage 72 (→ the storage tank 3) by absorbing and ejecting the raw material mixture solution.

Next, an action of the hydrogen production device 1 configured as above will be described. When the liquid feeding pump 9 is activated, the raw material mixture solution flows to circulate in the arrow A direction in Fig. 2. In the microwave device 5, the raw material mixture solution flows in the distribution tube 60 in the arrow B direction in Fig. 5. When the microwave device 5 is activated, a TM110 mode electromagnetic field is exited in the irradiation chamber 532 (in other words, the raw material mixture solution flowing in the distribution tube 60 is irradiated with a microwave).

As described above, the axis C2 of the distribution tube 60 is almost in line with the center line C1 which is a direction of the electric field generated in the irradiation chamber 532. Thus, the raw material mixture solution guided to the distribution tube 60 in the microwave device 5 first sufficiently absorbs the microwave when the raw material mixture solution flows through a portion on the upper side of the catalyst holding section 601 of the distribution tube 60. Thus, the temperature of the raw material mixture solution is quickly increased.

Subsequently, the raw material mixture solution flows in the catalyst holding section 601. As described above, the plurality of particulate members 12 and the plurality of solid catalysts 10 are held in the catalyst holding section 601. Thus, in the catalyst holding section 601, the flow of the raw material mixture solution is disturbed (agitated). In addition, the electric field distribution in the catalyst holding section 601 is not uniform due to the presence of the plurality of particulate members 12 and the plurality of solid catalysts 10, and the intensity of the electric field is reduced in average. Thus, in the catalyst holding section 601, the absorption of the microwave by the raw material mixture solution is reduced as compared to before flowing in the catalyst holding section 601. In other words, the raw material mixture solution passes through the catalyst holding section 601 while being agitated with the temperature increase suppressed. Accordingly, by increasing the temperature of the raw material mixture solution to a temperature around the lower limit of an appropriate temperature range before flowing in the catalyst holding section 601, the temperature of the raw material mixture solution can be kept in the appropriate temperature range during passing through the catalyst holding section 601. In addition, the opportunity of contact between substances that undergo a reaction in the raw material mixture solution and the opportunity of contact between the raw material mixture solution and the catalyst are also increased. As a result, a hydrogen production reaction can be promoted in the catalyst holding section 601 to efficiently produce hydrogen.

Subsequently, the raw material mixture solution and the produced hydrogen flow out from the lower end 60b of the distribution tube 60 and are guided (returned) via the return passage 72 to the storage tank 3. Then, the produced hydrogen is taken out from the hydrogen takeout port 32. The raw material mixture solution consumed by the production of hydrogen is appropriately replenished into the storage tank 3 from the supply and replenishment port 31. For example, a liquid level sensor may be provided in the storage tank 3, and when the liquid surface of the raw material mixture solution in the storage tank 3 is lowered to a first prescribed position, the raw material mixture solution may be replenished so that the liquid surface of the raw material mixture solution is increased to a second prescribed position which is higher than the first prescribed position. Thus, hydrogen can be continuously produced in the hydrogen production device 1.

Note that, when the alcohol is methanol, that is, when the raw material mixture solution is a mixture solution containing methanol, water, and a basic reagent, the device can be so configured that carbon dioxide produced together with hydrogen is discharged from the carbon dioxide discharge port 33 and methanol and water (aqueous methanol solution) is replenished from the supply and replenishment port 31.

In this embodiment, the raw material mixture solution corresponds to the "solution" in the present invention, the storage tank 3 corresponds to the "storage section" of the present invention, the fluid passage 7 and the liquid feeding pump 9 correspond to a "circulation means" of the present invention, the microwave device 5 corresponds to the "microwave irradiation means" of the present invention, the hydrogen takeout port 32 corresponds to a "first takeout unit" of the present invention, and the supply and replenishment port 31 corresponds to a "replenishing unit" of the present invention.

The hydrogen production device 1 according to this embodiment is configured to irradiate the raw material mixture solution (alcohol + water + basic reagent) passing through the catalyst holding section 601 with a microwave while circulating the raw material mixture solution between the storage tank 3 and the catalyst holding section 601 to cause the hydrogen production reaction. By circulating the raw material mixture solution, the raw material mixture solution repeatedly passes through the catalyst holding section 601 holding the plurality of solid catalysts 10. The microwave can quickly increase the temperature of the circulated (flowing) raw material mixture solution to a temperature suitable for the hydrogen production reaction. Thus, the hydrogen production device 1 according to this embodiment is capable of efficiently producing hydrogen.

In the hydrogen production device 1 according to this embodiment, the storage tank 3 has the supply and replenishment port 31, making it possible to appropriately replenish the raw material mixture solution. Thus, the hydrogen production device 1 according to this embodiment is capable of continuously producing hydrogen.

Furthermore, in the hydrogen production device 1 according to this embodiment, the distribution tube 60 including the catalyst holding section 601 is detachably attached to the cavity resonator 53 (the irradiation chamber 532) in the microwave device 5. Thus, in the hydrogen production device 1 according to this embodiment, the distribution tube 60 can be easily replaced and the catalyst (the plurality of solid catalysts 10) can be easily collected and replaced.

Note that, in this embodiment, the hydrogen production device 1 is configured to irradiate the raw material mixture solution (alcohol + water + basic reagent) passing through the catalyst holding section 601 with a microwave while circulating the raw material mixture solution between the storage tank 3 and the catalyst holding section 601 to cause the hydrogen production reaction. However, the present invention is not limited to this aspect. As described above, in the hydrogen production device 1, in place of the raw material mixture solution, a mixture solution of an organic compound and water, such as an aqueous alcohol solution (alcohol + water), or only an organic compound, such as only an alcohol, may be circulated between the storage tank 3 and the catalyst holding section 601. In this case, hydrogen can be continuously produced by appropriately replenishing the aqueous organic compound solution or only the organic compound. Note that, in this case, each of the aqueous organic compound solution and only the organic compound (that is, the organic compound itself, such as an alcohol) can correspond to the "solution" in the present invention, of course.

In this embodiment, the plurality of particulate members 12 are disposed above the plurality of solid catalysts 10. That is, the plurality of solid catalysts 10 and the plurality of particulate members 12 are held in the catalyst holding section 601 in the distribution tube 60. However, the present invention is not limited to this aspect. Only the plurality of solid catalysts 10 may be held in the catalyst holding section 601, or the plurality of particulate members 12 may be disposed above and below the plurality of solid catalysts 10, or the plurality of solid catalysts 10 and the plurality of particulate members 12 may be held in a mixed manner in the catalyst holding section 601.

In this embodiment, the distribution tube 60 is formed as a straight tube. However, the present invention is not limited to this aspect. The distribution tube 60 may have a spiral tube portion extending in a spiral form into the irradiation chamber 532 and all or a part of the spiral tube portion may constitute the catalyst holding section 601. In this case, the distribution tube 60 includes the spiral tube portion and a pair of straight tube portions each connected to one of both ends of the spiral tube portion. The spiral tube portion can be inserted into the through hole 533a formed in the top wall 533 constituting the cavity resonator 53 and the lid member 61 is attached to one of the straight tube portions. The other of the straight tube portions is inserted into the through hole of the distribution tube holding member 62. The absorption of the microwave by the raw material mixture solution is reduced in the case of passing through a spiral tube, as compared with the case of passing through a straight tube. Thus, with the distribution tube 60 including the spiral tube portion, the temperature of the raw material mixture solution can be kept at an appropriate temperature range for a longer period of time and the opportunity of contact (contact time) between the raw material mixture solution and the catalyst can be increased. As a result, hydrogen can be produced more efficiently.

In this embodiment, the raw material mixture solution flows downwardly from above through the distribution tube 60 (in the irradiation chamber 532). However, the present invention is not limited to this aspect, and the raw material mixture solution may flow upwardly from below through the distribution tube 60 (in the irradiation chamber 532). In this case, one end of the supply passage 71 is connected to the outlet 34 for the raw material mixture solution in the storage tank 3, and the other end of the supply passage 71 is connected to the lower end 60b of the distribution tube 60 in the microwave device 5. In addition, one end of the return passage 72 is connected to the upper end 60a of the distribution tube 60 in the microwave device 5, and the other end of the return passage 72 is connected to the inlet 35 for the raw material mixture solution in the storage tank 3. Furthermore, in the catalyst holding section 601, the plurality of particulate members 12 are mainly disposed below the plurality of solid catalysts 10. Also in this aspect, the same effect as in this embodiment is achieved.

Note that, although this embodiment is described basically by using the raw material mixture solution as a solution containing an organic compound, the present invention can be implemented in the same manner when the organic compound is replaced with an organic hydride or the like, of course.

In addition, in this embodiment, when the organic compound has a cyclic structure, the organic compound can be aromatized through production of hydrogen (dehydrogenation). Example of such organic compounds include saturated hydrocarbons, such as methylcyclohexane, cyclohexane, ethylcyclohexane, methoxycyclohexane, decalin, bicyclohexyl, and cyclohexylbenzene, and heterocyclic compounds, such as tetrahydrofuran (THF), tetrahydropyran (THP), tetrahydroquinoline, tetrahydroisoquinoline, piperidine, methylpiperidine, piperazine, methylpiperazine, and dimethylpiperazine.

Since hydrogen is produced from an organic compound in this embodiment, the hydrogen may further be subjected to intramolecular hydrogenation in an organic compound, or to hydrogenation of, for example, an unsaturated bond, such as a double bond, a triple bond, or an aromatic ring, or another compound having a structure to which hydrogen can be added, such as an azide group, a nitro group, a carbonyl group, an aromatic halogen, an epoxide, a benzyl ester, a benzyl ether, a benzyloxycarbonyl group (Cbz group), or a silyl-based protection group, for example, a trimethylsilyl group (TMS group) or a triethylsilyl group (TES group).

Furthermore, it is found from this embodiment that the present invention can be utilized for a vehicle off-gas cleanup application (production of NOx reduction components and soot flammable components), such as hydrogen production by cracking of a long CH chain in gasoline, gas oil, or the like, or hydrogen production from CH and water by steam reforming.

Examples of this embodiment will be described below. However, the following examples in no way limit the present invention.

### (Example 1) Production of Hydrogen from Mixture Solution Containing Methanol, Water, and Sodium Hydroxide

Methanol and water were mixed at a ratio by volume of 2:1 (20 mL : 10 mL) to prepare an aqueous methanol solution, and 4.64 g (116 mmol) of sodium hydroxide was added to the prepared aqueous methanol solution to prepare a raw material mixture solution. In the hydrogen production device 1 described above, the prepared raw material mixture solution (aqueous methanol solution + sodium hydroxide) was circulated at a liquid feeding rate of 0.3 to 0.5 mL/min. Thus, hydrogen was able to be taken out from the hydrogen takeout port 32.

### (Example 2) Production of Hydrogen from Mixture Solution Containing Isopropanol, Water, and Sodium Hydroxide

Isopropanol and water were mixed in a ratio by volume of 2:1 (120 mL : 60 mL) to prepare an aqueous isopropanol solution, and 1.6 g (40 mmol) of sodium hydroxide was added to the prepared aqueous isopropanol solution to prepare a raw material mixture solution. In the hydrogen production device 1, the prepared raw material mixture solution (aqueous isopropanol solution + sodium hydroxide) was circulated at a liquid feeding rate of about 1.0 mL/min. Thus, hydrogen was able to be taken out from the hydrogen takeout port 32.

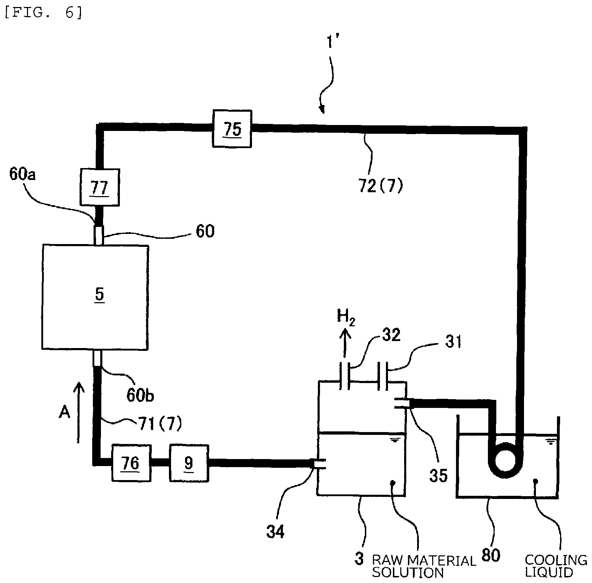

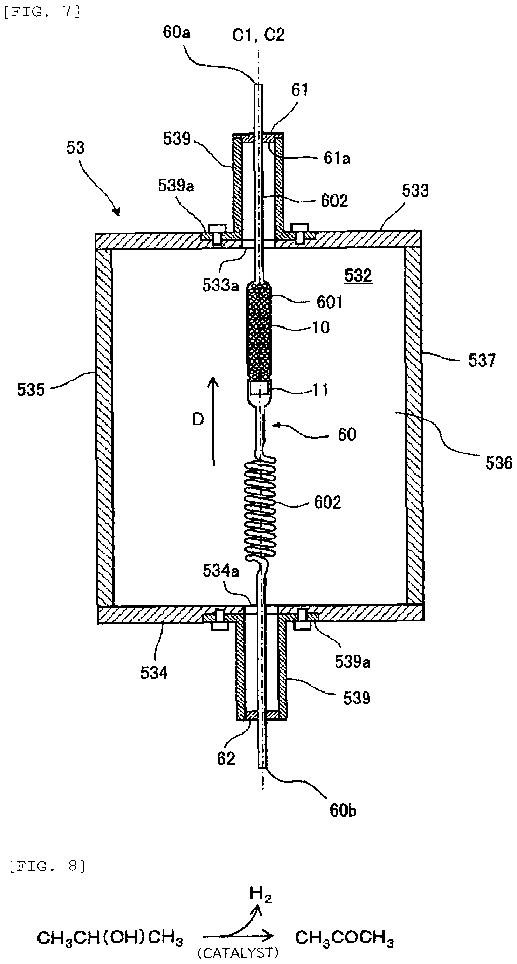

Next, the second embodiment of the present invention will be described with reference to Fig. 6 and Fig. 7. Fig. 6 is a schematic view of a configuration of a hydrogen production device according to this embodiment. Fig. 7 is a cross section illustrating an example of an installation state of a distribution tube into (an irradiation chamber of) a cavity resonator in a microwave device according to this embodiment.

Differences from the first embodiment described above will be described here.

In this embodiment, the hydrogen production device 1' produces hydrogen from a solution containing an organic compound (an alcohol) (hereinafter referred to as "raw material solution") .

The storage tank 3 constituting the hydrogen production device 1' is capable of storing a prescribed amount of the raw material solution. The storage tank 3 is provided with the supply and replenishment port 31 for supplying and replenishing the raw material solution into the storage tank 3 and the hydrogen takeout port 32 for taking out produced hydrogen.

The microwave device 5 constituting the hydrogen production device 1' is configured to irradiate a solution to be treated (the raw material solution corresponds to the solution to be treated here) flowing through the distribution tube 60 with a microwave.

In this embodiment, one end of the supply passage 71 is connected to the outlet 34 for the raw material solution in the storage tank 3 and the other end of the supply passage 71 is connected to the lower end 60b of the distribution tube 60 in the microwave device 5. One end of the return passage 72 is connected to the upper end 60a of the distribution tube 60 in the microwave device 5 and the other end of the return passage 72 is connected to the inlet 35 for the raw material solution in the storage tank 3. Then, as illustrated by the arrow D in Fig. 7, the raw material solution flows upwardly from below through the distribution tube 60 (in the irradiation chamber 532).

In this embodiment, the distribution tube 60 includes a spiral tube portion 602 below the catalyst holding section 601 of a straight tube form and above the lower end 60b (in other words, on the upstream side of the catalyst holding section 601). The spiral tube portion 602 extends in a spiral form in the irradiation chamber 532. Note that, although the particulate members 12 described above are omitted in this embodiment, the particulate members 12 may be held in the catalyst holding section 601 in the same manner as in the first embodiment.

In this embodiment, the liquid feeding pump 9 provided in the supply passage 71 circulates the raw material mixture solution in the order of the storage tank 3 → the supply passage 71 → the distribution tube 60 in the microwave device 5 (spiral tube portion 602 → the catalyst holding section 601) → the return passage 72 (→ the storage tank 3) by absorbing and ejecting the raw material solution.

In this embodiment, a back pressure valve 75 is provided in the middle of the return passage 72. A pressure gauge 76 for grasping the back pressure in the reaction system is provided on the downstream side of the liquid feeding pump 9 in the supply passage 71. By changing the opening of the back pressure valve 75, the back pressure in the reaction system can be changed. The back pressure in the reaction system is not particularly limited as long as it falls in the range where the reaction proceeds, and is preferably 0 to 10 MPa. In this embodiment, the reaction is a reaction in which hydrogen is generated, a lower back pressure is preferred since the reaction proceeds quickly. However, when air bubbles are generated in the reaction tube, it may be difficult to control the microwave.

In this embodiment, a temperature sensor 77 is provided at one end of the return passage 72 (around the upper end 60a of the distribution tube 60). The temperature sensor 77 is for grasping the temperature of the solution to be treated immediately after passing through the catalyst holding section 601.

In this embodiment, the hydrogen production device 1' includes a cooling device 80 for cooling the solution to be treated flowing through the return passage 72. The cooling device 80 includes, for example, a storage section for storing a cooling liquid. In the cooling device 80, by allowing the cooling liquid to absorb heat of the solution to be treated flowing through the return passage 72, the temperature of the solution to be treated can be decreased. In the cooling device 80, the temperature of the cooling liquid can be controlled to a desired temperature by a controlling means not shown. Note that the cooling device 80 is not limited to a liquid-cooling type and may be an air-cooling type.

Fig. 8 is a formula illustrating an example of a hydrogen production reaction in the hydrogen production device and the hydrogen production method according to this embodiment, in which the alcohol constituting the raw material solution is isopropanol.

As shown in Fig. 8, when the alcohol constituting the raw material solution is isopropanol, hydrogen and acetone may be produced in a ratio of 1:1. It is also understood that hydrogen (and acetone) can be continuously produced by appropriately replenishing the isopropanol consumed with the production of hydrogen (and acetone) via the supply and replenishment port 31 in the hydrogen production reaction shown in Fig. 8. Here, in the hydrogen production reaction shown in Fig. 8, a carbonyl compound produced with the production of hydrogen is acetone. In other words, in the hydrogen production reaction shown in Fig. 8, a ketone produced with the production of hydrogen is acetone.

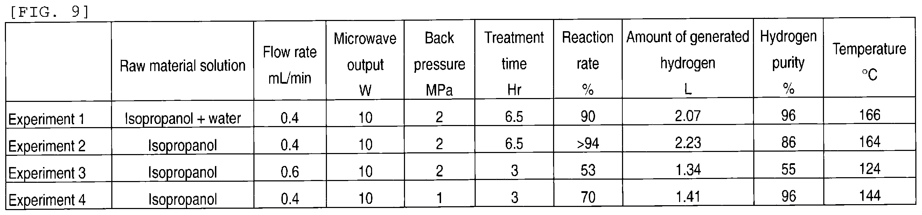

Four hydrogen production experiments were carried out using the hydrogen production device 1' of this embodiment. The results of the four experiments are shown in Fig. 9 (Experiments 1 to 4) . Here, in Experiment 1, a 20 mol% aqueous isopropanol solution (solution containing isopropanol and water) was used as the raw material solution. In Experiments 2 to 4, isopropanol was used as the raw material solution.

### [Common Items of Experiments 1 to 4]

The total amount of the raw material solution present in the system of the hydrogen production device 1' (in the system including the storage tank 3, the fluid passage 7, and the distribution tube 60) was 6.9 mL immediately before the start of each experiment. In the catalyst holding section 601 of glass, 80 mg of a Pt/carbon bead catalyst produced in Production Example 1 was placed as the solid catalyst 10. The flow rate shown in Fig. 9 corresponds to the liquid feeding rate of the raw material solution in each experiment. The microwave output shown in Fig. 9 is the output of the microwave in the microwave device 5. The back pressure shown in Fig. 9 is the indication value (measurement value) of the pressure gauge 76. The treatment time shown in Fig. 9 is the time in which production of hydrogen was carried out in each experiment. The reaction rate shown in Fig. 9 indicates a proportion (substance quantitative ratio) of isopropanol used in production of hydrogen in the above treatment time based on isopropanol in the raw material solution in each experiment. For example, the reaction rate of 90% in Experiment 1 means that 90% of isopropanol present at the beginning of the experiment was used in the production of hydrogen in 6.5 hours (the above treatment time) . The reaction rate can be grasped, based on the hydrogen production reaction shown in Fig. 8, by identifying the substance quantitative ratio of isopropanol and acetone remaining after the treatment time in each experiment by ¹H NMR. The amount of generated hydrogen shown in Fig. 9 indicates the amount of hydrogen produced in the above treatment time (in other words, the amount of hydrogen generated in the treatment time) in each experiment, which is specifically the amount of hydrogen taken out from the hydrogen takeout port 32 in the treatment time. The hydrogen purity shown in Fig. 9 indicates the purity of hydrogen produced in the treatment time (the purity of hydrogen taken out from the hydrogen takeout port 32 in the treatment time) in each experiment, which is the ratio of the amount of hydrogen relative to the total amount of gas taken out from the hydrogen takeout port 32 in the treatment time. Gas chromatography can be used for identification of the amount of generated hydrogen and the hydrogen purity shown in Fig. 9. The temperature shown in Fig. 9 is the highest value of the temperatures measured with the temperature sensor 77.

### [Production Example 1]

Into a 5L beaker, 2.4 L of pure water and 16 g of soda ash (Na₂CO₃) were put at 30°C and stirred, 16 g of potassium chloroplatinate (K₂PtCl₆) was dissolved, 110 g of an activated carbon bead (A-BAC-MP, manufactured by KUREHA CORPORATION, average particle size based on weight: 500 µm, D50 particle size: 456 µm, specific surface area: 1291 m²/g, spherical) was put, and then the mixture was heated to 90°C, followed by stirring for 30 minutes. In this solution, 34 mL of 33% sodium formate (HCOONa) was added and the mixture was stirred for 30 minutes for catalyst supporting. The resultant was washed with 25 L of pure water by decantation, followed by filtration and drying at 80°C overnight, thereby obtaining a Pt/carbon bead catalyst containing 5% by weight of platinum. Note that the D50 particle size and the specific surface area were measured as follows.

### <D50 Particle Size>

Measurement principle: laser diffraction and scattering method (wet measurement)
Measurement device: MT3300EXII, manufactured by Microtrac <Specific Surface Area>
Measurement principle: nitrogen gas adsorption method
Measurement device: ASAP 2420, manufactured by Micromeritics

### [Experiment 1]

In Experiment 1, a 20 mol% aqueous isopropanol solution (solution containing isopropanol and water) was used as the raw material solution. In Experiment 1, the raw material solution was circulated while the flow rate was set to 0.4 mL/min, the microwave output was to 10 W, the back pressure was to 2 MPa, and the treatment time was to 6.5 hours, as setting conditions. Under the conditions, hydrogen production was performed using the hydrogen production device 1' . Then, 2.07 L of hydrogen was taken out from the hydrogen takeout port 32, and the purity of the hydrogen taken out was 96%. In Experiment 1, the reaction rate was 90%. In Experiment 1, the temperature of the raw material solution immediately after passing through the catalyst holding section 601 reached 166°C.

### [Experiment 2]

In Experiment 2, isopropanol was used as the raw material solution. In Experiment 2, the raw material solution was circulated while the flow rate was set to 0.4 mL/min, the microwave output was to 10 W, the back pressure was to 2 MPa, and the treatment time was to 6.5 hours, as setting conditions. Under the conditions, hydrogen production was performed using the hydrogen production device 1' . Then, 2.23 L of hydrogen was taken out from the hydrogen takeout port 32, and the purity of the hydrogen taken out was 86%. In Experiment 2, the reaction rate was 94% or more. In Experiment 2, the temperature of the raw material solution immediately after passing through the catalyst holding section 601 reached 164°C.

### [Experiment 3]

In Experiment 3, isopropanol was used as the raw material solution. In Experiment 3, the raw material solution was circulated while the flow rate was set to 0 . 6 mL/min, the microwave output was to 10 W, the back pressure was to 2 MPa, and the treatment time was to 3 hours, as setting conditions. Under the conditions, hydrogen production was performed using the hydrogen production device 1'. Then, 1.34 L of hydrogen was taken out from the hydrogen takeout port 32, and the purity of the hydrogen taken out was 55%. In Experiment 3, the reaction rate was 53%. In Experiment 3, the temperature of the raw material solution immediately after passing through the catalyst holding section 601 reached 124°C.

### [Experiment 4]

In Experiment 4, isopropanol was used as the raw material solution. In Experiment 4, the raw material solution was circulated while the flow rate was set to 0.4 mL/min, the microwave output was to 10 W, the back pressure was to 1 MPa, and the treatment time was to 3 hours, as setting conditions. Under the conditions, hydrogen production was performed using the hydrogen production device 1' . Then, 1.41 L of hydrogen was taken out from the hydrogen takeout port 32, and the purity of the hydrogen taken out was 96%. In Experiment 4, the reaction rate was 70%. In Experiment 4, the temperature of the raw material solution immediately after passing through the catalyst holding section 601 reached 144°C.

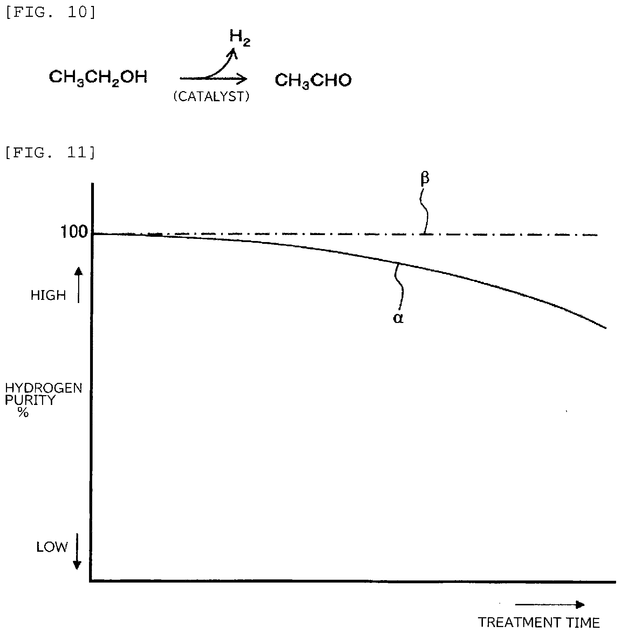

Fig. 10 is a formula illustrating another example of a hydrogen production reaction in the hydrogen production device and the hydrogen production method according to this embodiment, in which the alcohol constituting the raw material solution is ethanol.

As shown in Fig. 10, when the alcohol constituting the raw material solution is ethanol, hydrogen and acetaldehyde can be produced at a ratio of 1: 1. It can also be understood that hydrogen (and acetaldehyde) can be continuously produced by appropriately replenishing ethanol consumed with the production of hydrogen (and acetaldehyde) via the supply and replenishment port 31 in the hydrogen production reaction shown in Fig. 10. Here, in the hydrogen production reaction shown in Fig. 10, a carbonyl compound produced with the production of hydrogen is acetaldehyde. In other words, in the hydrogen production reaction shown in Fig. 10, an aldehyde produced with the production of hydrogen is acetaldehyde. Also when the alcohol constituting the raw material solution is ethanol, it is possible to produce hydrogen using the hydrogen production device 1' to take out hydrogen from the hydrogen takeout port 32.

In this embodiment, the reaction system is neutral. However, in the same manner as in the first embodiment, the raw material solution may further contain a basic reagent, and in this case, the reaction system may be basic.

Fig. 11 is a graph illustrating a relationship between the time in which hydrogen production is performed by the hydrogen production device 1' (treatment time) and the purity of hydrogen taken out from the hydrogen takeout port 32 (hydrogen purity) . Here, the alcohol in the raw material solution is isopropanol.

The present inventors have found that, when an operation in which the raw material solution passing through the catalyst holding section 601 is irradiated with a microwave while circulating the raw material solution between the storage tank 3 and the catalyst holding section 601 to cause the hydrogen production reaction is continued, the purity of hydrogen taken out from the hydrogen takeout port 32 tends to decrease with the treatment time as shown by the solid line α in Fig. 11. In other words, the present inventors have found that gas other than hydrogen taken out from the hydrogen takeout port 32 tends to increase with the treatment time. The present inventors have also found that the gas other than hydrogen may contain carbon monoxide, carbon dioxide, and the like.

The present inventors have also found that, when an operation in which the raw material solution passing through the catalyst holding section 601 is irradiated with a microwave while merely supplying the raw material solution from the storage tank 3 into the catalyst holding section 601 without circulating the raw material solution between the storage tank 3 and the catalyst holding section 601 to cause the hydrogen production reaction is continued, even if the treatment time elapses, the purity of hydrogen taken out of the reaction system is maintained at a high value (almost at 100%) (see dashed line (3 in Fig. 11).

Based on the above findings, the present inventors have found that acetone produced with the production of hydrogen (see Fig. 8) has an influence on generation of the gas other than hydrogen to no small extent. Based on the findings, the present inventors propose the following embodiment (third embodiment).

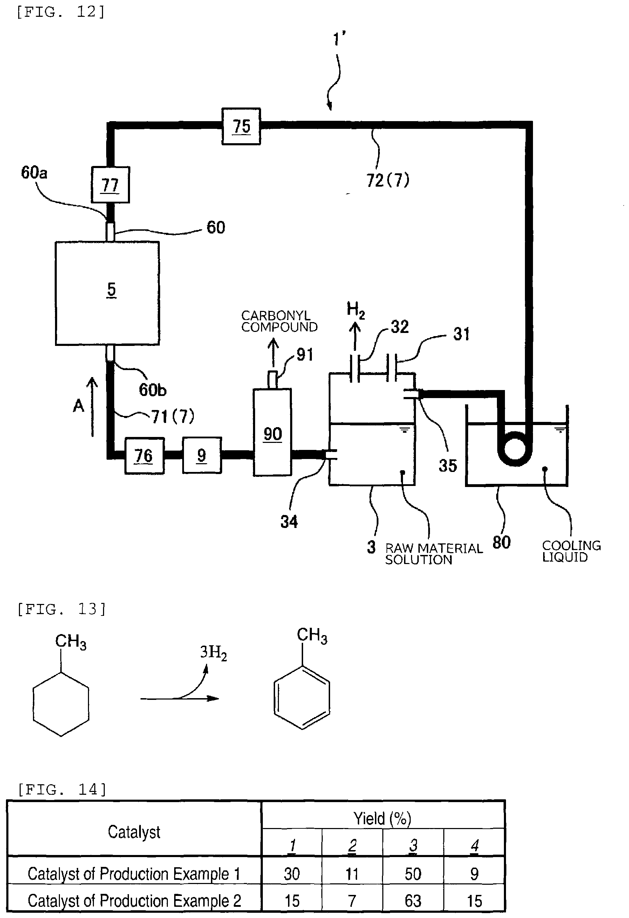

Fig. 12 is a schematic diagram illustrating a configuration of a hydrogen production device according to the third embodiment of the present invention.

Differences from the second embodiment will be described.

In this embodiment, the hydrogen production device 1' further includes a separation device 90. The separation device 90 is provided on the upstream side of the liquid feeding pump 9 in the supply passage 71. The separation device 90 can achieve an action of separating a carbonyl compound which is produced with the production of hydrogen and is mixed in the raw material solution, from the raw material solution. In the separation device 90, the carbonyl compound is separated from the raw material solution by distillation using the difference between the boiling point of the raw material solution and the boiling point of the carbonyl compound. The separation device 90 is provided with a takeout port 91 for taking out the carbonyl compound separated from the raw material solution. Here, the takeout port 91 corresponds to a "second takeout unit" of the present invention.

When the alcohol constituting the raw material solution is isopropanol, acetone (a carbonyl compound, a ketone) is separated from the raw material solution in the separation device 90, and the acetone (carbonyl compound, ketone) can be taken out from the takeout port 91. When the alcohol constituting the raw material solution is ethanol, acetaldehyde (a carbonyl compound, an aldehyde) is separated from the raw material solution in the separation device 90, and the acetaldehyde (carbonyl compound, aldehyde) can be taken out from the takeout port 91.

According to this embodiment, the carbonyl compound which is produced with the production of hydrogen and is mixed in the raw material solution can be separated from the raw material solution by the separation device 90 and can be taken out from the takeout port 91. Thus, even when an operation in which the raw material solution passing through the catalyst holding section 601 is irradiated with a microwave while circulating the raw material solution between the storage tank 3 and the catalyst holding section 601 to cause the hydrogen production reaction is continued for a long period of time, the purity of hydrogen taken out from the hydrogen takeout port 32 can be maintained at a high value.

Note that, although the hydrogen takeout port 32 for taking out the produced hydrogen is provided in the storage tank 3 in this embodiment, the hydrogen takeout port 32 may be provided in the separation device 90, alternatively. In this case, the carbonyl compound and hydrogen can be separated from the raw material solution by distillation using the difference among the boiling point of the raw material solution, the boiling point of the carbonyl compound, and the boiling point of hydrogen in the separation device 90.

Although the separation device 90 is provided on the upstream side of the liquid feeding pump 9 in the supply passage 71 in this embodiment, the installation position of the separation device 90 is not limited to this aspect, and any position may be selected. In addition, the separation device 90 may be applied to the first embodiment, of course.

The installation position of the cooling device 80 in the second and third embodiments is not limited to the position illustrated in Fig. 6 and Fig. 12, and any position may be selected. The cooling device 80 may be configured to cool the raw material solution stored in the storage tank 3. The cooling device 80 may be applied to the first embodiment, of course.

The raw material mixture solution in the first embodiment and the raw material solution in the second and third embodiments may contain not only one alcohol alone but may contain two or more alcohols . The alcohol in the raw material mixture solution may be, for example, at least one of methanol, ethanol, and isopropanol. The alcohol in the raw material solution may be, for example, at least one of methanol, ethanol, and isopropanol.

Fig. 13 is a formula illustrating an example (forth embodiment) of a hydrogen production reaction in the hydrogen production device and the hydrogen production method according to this embodiment, in which hydrogen is produced from a solution containing an organic compound (organic hydride). Specifically, the organic hydride is methylcyclohexane.

As shown in Fig. 13, when the organic hydride constituting the raw material solution is methylcyclohexane, hydrogen and toluene may be produced at a ratio of 3:1. It can be understood that hydrogen (and toluene) can be continuously produced by appropriately replenishing methylcyclohexane consumed with the production of hydrogen (and toluene) via the supply and replenishment port 31 in the hydrogen production reaction shown in Fig. 13. Here, in the hydrogen production reaction shown in Fig. 13, methylcyclohexane is aromatized into toluene by the production of hydrogen.

Two hydrogen production experiments were carried out using the hydrogen production device 1' of this embodiment.

### [Common Items in Experiments 5 to 6]

Items are the same as the common items in Experiments 1 to 4 except for the raw material solution.

### [Experiment 5]

In Experiment 5, methylcyclohexane was used as the raw material solution. In Experiment 5, the raw material solution was fed while the flow rate was set to 0.5 mL/min, the microwave output was to 10 W, the back pressure was to 0 MPa, and the treatment time was to 3.5 hours, as setting conditions. Under the conditions, hydrogen production was performed using the hydrogen production device 1'. Then, 6.65 L of hydrogen was taken out from the hydrogen takeout port 32, and the purity of the hydrogen taken out was 99.8%. In Experiment 5, the reaction rate was 95%. In Experiment 5, the temperature of the raw material solution immediately after passing through the catalyst holding section 601 reached 122°C. Toluene was collected from the takeout port.

### [Experiment 6]

In Experiment 6, methylcyclohexane was used as the raw material solution. In Experiment 6, the raw material solution was fed while the flow rate was set to 0.8 mL/min, the microwave output was to 10 W, the back pressure was to normal pressure, and the treatment time was to 3.5 hours, as setting conditions. Under the conditions, hydrogen production was performed using the hydrogen production device 1'. Then, 5.32 L of hydrogen was taken out from the hydrogen takeout port 32, and the purity of the hydrogen taken out was 99.7%. In Experiment 6, the reaction rate was 87%. In Experiment 6, the temperature of the raw material solution immediately after passing through the catalyst holding section 601 reached 125°C. Toluene was collected from the takeout port.

### [Experiment 7]

Hydrogen production was performed in the same manner as in Experiment 5 except that 200 mL of methylcyclohexane (1.57 mol) was fed in a liquid feeding time of 15 hours. Then, the catalyst was not deactivated and 38.7 L of hydrogen gas was obtained at a purity of 99.9%. In this time, the catalyst catalyzed the dehydrogenation reaction 70,000 times or more ([methylcyclohexane → methylcyclohexene + H₂] was counted as once) per atom of the catalyst metal, and it is expected that a large amount of hydrogen gas can be produced with a small amount of a catalyst metal.

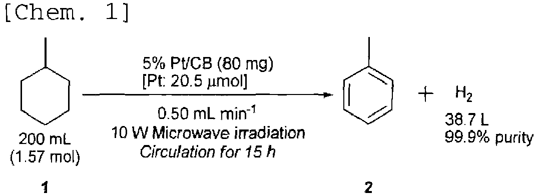

### [Comparative Experiment 1]

In Comparative Experiment 1, methylcyclohexane was used as the raw material solution. In Comparative Experiment 1, the raw material solution was fed while the flow rate was set to 0.5 mL/min, the microwave output was to 10 W, the back pressure was to normal pressure, an activated carbon bead (BAC-MP, manufactured by KUREHA CORPORATION, average particle size: 500µm, specific surface area: 1200 m²/g, spherical) with no noble metal supported was used in place of the Pt/carbon bead catalyst, and the treatment time was set to 3.5 hours, as setting conditions. Under the conditions, hydrogen production was performed using the hydrogen production device 1' . Then, little hydrogen was taken out from the takeout port 32. In Comparative Experiment 1, the temperature of the raw material solution immediately after passing through the catalyst holding section 601 reached 109°C.

### [Comparative Experiment 2]

In Comparative Experiment 2, methylcyclohexane was used as the raw material solution. In Comparative Experiment 2, a 10% Pt-supporting activated carbon (product name: 10% Pt-C(W)K type catalyst, manufactured by N. E. Chemcat Corporation, average particle size: 30 µm, powdery) was used in place of the Pt/carbon bead catalyst. The solution was not able to be fed due to clogging.

### [Experiment 8]

A reaction was performed in the same manner as in Experiment 5 except that the liquid feeding time was 5.5 hours and ethylcyclohexane was used as the raw material solution. Then, ethylbenzene was obtained at a yield of 78%.

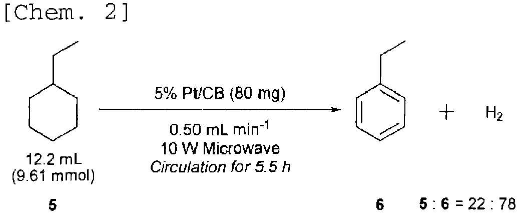

### [Experiment 9]

The liquid feeding time was 4 hours and a 0 . 5 mol/L of solution of decalin in toluene was used as the raw material solution in Experiment 8. Hydrogen production was performed in the same manner as in Experiment 8 except that the back pressure was 0 . 5 MPa. Then, decalin was obtained at a yield of 77%.

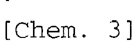

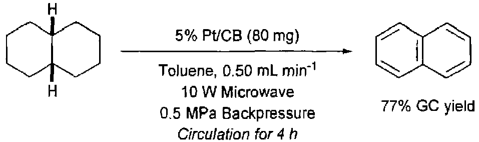

### [Experiment 10]

The liquid feeding time was 2 hours and a 0.5 mol/L solution of 1,2,3,4-tetrahydroisoquinoline in toluene was used as the raw material solution in Experiment 9. A reaction was performed in the same manner as in Experiment 9. Then, isoquinoline was obtained at a yield of 95%. Aromatization of a heterocyclic compound containing a nitrogen atom which may act as a catalyst poison also proceeded efficiently.

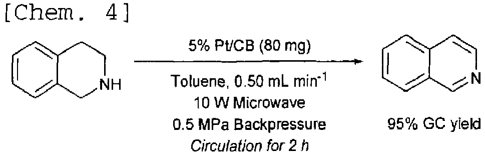

### [Experiment 11]

The liquid feeding time was 3.5 hours and a 0.5 mol/L solution of 4-methylpiperidine in toluene was used as the raw material solution in Experiment 9. A reaction was performed in the same manner as in Experiment 9. Then, 4-methylpyridine was obtained at a yield of 78%.

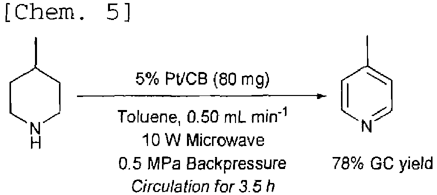

### [Production Example 2]

A Pt/carbon bead catalyst containing 5% by weight of platinum was obtained by a treatment in the same manner as in Production Example 1 except that the activated carbon bead in Production Example 1 was changed to another activated carbon bead (A-BAC-SP, manufactured by KUREHA CORPORATION, average particle size based on weight: 400 µm or less, D50 particle size: 328 µm, specific surface area: 1286 m²/g, spherical) . The D50 particle size and the specific surface area were measured in the same manner as in Production Example 1.

### [Experiment 12]

A reaction was performed in the same manner as in Experiment 9 except that the catalyst in Production Example 1 or Production Example 2 was used, the liquid feeding time was 10 minutes in a single pass, the raw material solution was a 0.5 mol/L solution of bicyclohexyl in toluene in Experiment 9, and the back pressure was 1.5 MPa. The yields of the compounds after the reactions were shown in Fig. 14.

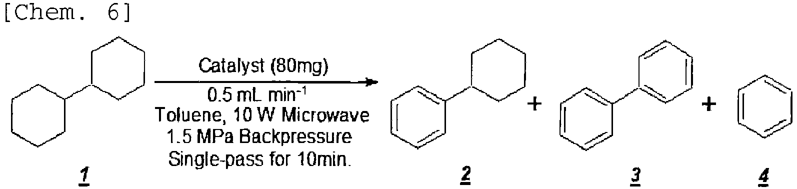

All of Compounds 2, 3, and 4 in the reaction formula which were practically measured by gas chromatography were obtained through dehydrogenation of Compound 1 which was a reactant, and it was found that the catalyst in Production Example 1 and the catalyst in Production Example 2 showed an excellent hydrogen production ability. The catalyst in Production Example 2, which used a carrier having a smaller particle size than that in Production Example 1, resulted in a high yield of biphenyl and benzene which were produced through dehydrogenation of bicyclohexyl which was a reactant. Thus, it was found that Production Example 2 showed an excellent hydrogen production ability.

As can be seen from the above, the embodiment merely shows an example of the present invention, and the present invention encompasses, in addition to the aspects directly illustrated by the embodiments described herein, a variety of modifications and variations made by a person skilled in the art in the scope of the claims, of course.

### Reference Signs List

1,1': hydrogen production device, 3: storage tank, 5: microwave device, 7: fluid passage, 9: liquid feeding pump, 10: solid catalyst, 12: particulate member, 31: supply and replenishment port, 32: hydrogen takeout port, 51: microwave generator, 52: waveguide, 53: cavity resonator, 54: controller, 60: distribution tube, 71: supply passage, 72: return passage, 80: cooling device, 90: separation device, 91: takeout port, 532: irradiation chamber, 601: catalyst holding section, 602: spiral tube portion

## Claims

1. A device comprising:
a storage section in which a solution containing an organic compound can be stored;
a catalyst holding section in which a solid catalyst is held; and
a microwave irradiation means configured to irradiate the solution passing through the catalyst holding section with a microwave,
the solid catalyst being a molded catalyst containing a noble metal supported on a carrier that has an average particle diameter larger than 100 µm.

2. A device according to claim 1, wherein the carrier comprises carbon.

3. A device according to claim 1 or 2, wherein the carrier has a specific surface area of 50 to 2000 m²/g.

4. A device according to any one of claims 1 to 3, wherein the organic compound is an alcohol or an organic hydride.

5. A device according to claim 4, wherein the alcohol is isopropanol.

6. A device according to claim 4, wherein the organic hydride is methylcyclohexane.

7. A device according to any one of claims 1 to 6, which is for hydrogen generation.

8. A device according to any one of claims 1 to 6, which is for aromatization.

9. A device according to any one of claims 1 to 6, which is for hydrogenation.

10. A catalyst for microwave irradiation, the catalyst being a molded catalyst containing a noble metal supported on a carrier that has an average particle diameter larger than 100 µm.

11. A method for producing hydrogen, the method comprising irradiating a solution containing an organic compound, the solution passing through a catalyst holding section that holds a solid catalyst, with a microwave, the solid catalyst being a molded catalyst containing a noble metal supported on a carrier that has an average particle diameter larger than 100 µm.

12. The method for producing hydrogen according to claim 11, wherein the device according to any one of claims 1 to 6 is used.

13. An aromatization method, the method comprising irradiating a solution containing an organic compound, the solution passing through a catalyst holding section that holds a solid catalyst, with a microwave, the solid catalyst being a molded catalyst containing a noble metal supported on a carrier that has an average particle diameter larger than 100 µm.

14. The aromatization method according to claim 13, wherein the device according to any one of claims 1 to 6 is used.

15. A hydrogenation method, the method comprising irradiating a solution containing an organic compound, the solution passing through a catalyst holding section that holds a solid catalyst, with a microwave, the solid catalyst being a molded catalyst containing a noble metal supported on a carrier that has an average particle diameter larger than 100 µm.

16. The hydrogenation method according to claim 15, wherein the device according to any one of claims 1 to 6 is used.
